**Europäisches Patentamt**

(19) **European Patent Office**

**Office européen des brevets**

(11) Publication number : **0 339 748 B1**

(12) **EUROPEAN PATENT SPECIFICATION**

(45) Date of publication of patent specification :
08.07.92 Bulletin 92/28

(51) Int. Cl.⁵ : **B01J 8/02,** C07D 301/10, C07D 301/08

(21) Application number : **89201070.3**

(22) Date of filing : **25.04.89**

(54) Process for the preparation of ethylene oxide.

(30) Priority : **27.04.88 GB 8810006**

(43) Date of publication of application :
02.11.89 Bulletin 89/44

(45) Publication of the grant of the patent :
08.07.92 Bulletin 92/28

(84) Designated Contracting States :
**BE DE FR GB IT NL**

(56) References cited :
**EP-A- 0 082 609**
**EP-A- 0 266 015**
**GB-A- 2 204 055**
**US-A- 1 835 827**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Van Dongen, Franciscus Gerardus**
**Badhuisweg 3**
**NL-1031 CM Amsterdam (NL)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of ethylene oxide by reacting ethylene with molecular oxygen in the presence of a silver-containing catalyst by passing a gas mixture of ethylene and oxygen through a reaction zone containing catalyst particles.

Processes for the conversion of ethylene and oxygen into ethylene oxide are known. As such conversions are highly exothermic processes, it will be appreciated that usually means have to be used for the removal of heat from the reaction zone. For instance, a suitable reactor is a multitube reactor in which a cooling medium flows through the spaces between the tubes. The tubes are filled with suitable catalyst particles. Ethylene and oxygen flow in downward direction through the tubes, and the ethylene oxide is removed from the bottompart of the reactor.

Nowadays there is an increasing demand for larger capacity equipment, not only because all (chemical) processes are carried out on ever larger scale but also because certain processes are increasingly used. In particular the synthesis of ethylene oxide by conversion of ethylene and oxygen, attracts increasing interest.

Upscaling of a reactor of the above multitube type to increase the capacity thereof may, however, have a severe adverse influence on the efficiency, particularly if the reactor is used for carrying out highly exothermic reactions. In these highly exothermic reactions the heat liberated has to be removed continuously to avoid undesirably high temperatures which might cause a sharp increase of the rate of reaction (possibly followed by deactivation of the catalyst) and/or the occurrence of unwanted runaway reactions. The aim of continuous heat removal, in exothermic reactions means with respect to tube type reactors that the tubes forming the reaction zones must have a relatively small cross-sectional area with a heat transfer medium circulated around the outer surfaces of the tubes. If the cross-sectional areas of the reaction zones are large, the middle parts of the zones are too far away from the heat transfer medium at the outside of said zones and hence tend to experience undesirable temperature increases or temperature drops. Increase of the capacity of a tube type reactor should therefore be accomplished by an increase of the number of tubes rather than by an increase of the tube diameters. The use of a large number of tubes enclosed in a necessarily large diameter reactor vessel poses, however, a number of problems, viz. firstly it becomes difficult to achieve uniform distribution of heat transfer medium over the full diameter of the reactor vessel, secondly uniform distribution of fluids over the various tubes becomes more difficult. A uniform distribution of heat transfer medium along the tubes is required to obtain a reaction product with a predetermined constituency and to prevent stresses in the bundles of tubes due to temperature differences.

An object of the present invention is to overcome the above problems encountered with increasing the capacity of tube type reactors suitable for carrying out highly exothermic reaction as the catalytic preparation of ethylene oxide.

It has now been found that the catalytic preparation of ethylene oxide very suitably may be carried out in a reactor comprising a catalyst bed wherein one or more helical wound cooling tubes have been installed, the reactor being maintained at conversion conditions.

The use of this type of reactor does not lead to the problem encountered with the increase of the capacity of a multitube reactor, especially the uniform distribution of heat transfer medium over the full diameter of the reaction vessel and along the tubes. Also a higher heat transfer of the process side is obtained, and no use has to be made of very large diameter tube sheets. Another advantage is that the thickness of the wall of the reactor is determined by the process pressure and not by the pressure of the cooling medium, as the process pressure is usually lower than the pressure of the cooling medium. The helical wound cooling tubes does not give large expansion problems, which makes the reactor less sensitive to temperature differences between the tubes and the reactor wall. Further, catalyst loading and unloading is easier in this type of reactor than it is in a multitube reactor.

The process of the present invention, therefore, relates to a process for the preparation of ethylene oxide by reacting ethylene with molecular oxygen in the presence of a silver-containing catalyst, which comprises passing a gas mixture of ethylene and oxygen through a reaction zone containing the catalyst particles, while removing heat from the reaction zone by a cooling medium which flows through the reaction zone via one or more helical patterns, each pattern containing one or more helices.

To obtain the helical patterns of the cooling medium helical wound tubes or tube bundles are used. Preferably a cylindrical reaction vessel is used provided with one or more helical wound tubes or tube bundles, each tube bundle comprising two or more helical wound tubes of substantially the same dimensions, and situated in a concentric ring or a number of concentric rings around the central axis of the reaction vessel. Thus, the cooling medium flows via one or more helical patterns situated concentrically around the central axis, each pattern containing one or more helices. When two or more concentric tubes or tube bundles are used, the screw-direction of the helices of two adjacent tubes or tube bundles are preferably opposite to each other. When two or more tube bundles are used it is preferred to use an increasing number of helical wound tubes in bundles situated at a larger distance from the central tube, and to maintain substantially the same length for each tube.

The helical flowing pattern of the cooling medium enables the ratio heat-exchanger surface/reactor volume to be varied over a large range. The tube diameter may be varied as well as the distance between two layers of tubing in both the axial and the radial direction. The diameter of the cooling tubes is suitably chosen between 4 and 55 mm, especially between 10 and 35 mm. The distance between two adjacent rings of tubes or tube bundles (distance in the radial direction) is suitably chosen between 10 and 50 mm, especially between 15 and 25 mm, and the distance between two adjacent coils lying in a concentric ring (distance in the axial direction) is suitably chosen between 10 and 200 mm, especially between 10 and 50 mm. The helical wound tube bundles make it possible to use hemispheric tube sheet designs, thus avoiding the less suitable flat tube sheets.

The heat exchange tubes are preferably spaced in such a manner in the reaction zone(s) that an optimal temperature profile is attained therein in radial direction. Moreover, each group (e.g. concentric ring) of heat exchange tubes may be in communication with separate cooling fluid in- and outlet means which can be operated independently of other groups of heat exchange tubes in order to attain optimal control over the temperature profile in the reaction zone(s).

Water or hydrocarbons are usually used as cooling medium. The water evaporates at least partially in the tubes. This enables the heat of reaction to be removed from the reaction zone by producing steam. Hydrocarbons with at least 10 carbon atoms are also preferred, e.g. isododecane. Other cooling media for instance are biphenyl, thermal oils and kerosene.

The feed referred to hereinabove contains as major components ethylene and oxygen; in addition said feed may contain ethane, nitrogen, and a very minor amount of vinylchloride (e.g. the latter between 1 and 10 parts per million).

The present process is preferably carried out at a temperature in the range of from 200 to 285 °C, a total pressure in the range of from 1 to 60 bar abs. and a gas hourly space velocity in the range of from 2000 to 8000 $h^{-1}$. Particularly preferred process conditions for the preparation of ethylene oxide include a temperature from 220-260 °C, a pressure from 5-40 bar abs. and a gas hourly space velocity from 2500-6000 $h^{-1}$. In the gaseous feed the ethylene/oxygen molar ratio is preferably from 2:1 to 4:1.

The silver-containing catalyst, used in the process for the reaction of ethylene and oxygen to ethylene oxide, is generally on an alpha-alumina carrier and additionally may contain an alkali metal(-oxide) promoter. The silver is generally present in an amount of 2 to 25% by weight, calculated on the weight of the total catalyst. The alkali metal promoter may be present in an amount of 50 to 5000 parts per million, based on the total weight of the catalyst. Preferred is a cesium promoter, but other alkali metals may be used as well.

The catalysts are preferably employed in the present process in the form of spherical, cylindrical or lobed particles with a diameter from 0.1-15 mm, and in particular from 0.5-5 mm. The catalyst particles can be prepared by a variety of methods known in the art, such as disclosed in GB-A 1,413,251.

The process according to the invention can also be employed with silver catalysts comprising as promoter alkali metals and other traces of elements, e.g. rhenium, supported on an inert carrier, preferably an alpha-alumina carrier. Preferably the amount of rhenium ranges from 0.01 to 15 millimol rhenium per kg of catalyst, more preferably from 0.2 to 5 millimol rhenium per kg of catalyst. The amount of alkali ranges from 10 to 3000 parts per million per kg of catalyst, preferably from 50 to 1000 parts per million per kg of catalyst. Additionally the catalyst may comprise sulphur. The alpha-alumina carrier may have a surface area in the range of from 0.01 to 10 $m^2/g$, preferably of from 0.05 to 5 $m^2/g$, more preferably of from 0.1 to 3 $m^2/g$. The amount of silver is preferably in the range of from 2 to 25% by weight, calculated on the weight of the total catalyst.

## Claims

1. Process for the preparation of ethylene oxide by reacting ethylene with molecular oxygen in the presence of a silver-containing catalyst, which comprises passing a gas mixture of ethylene and oxygen through a reaction zone containing the catalyst particles, while removing heat from the reaction zone by a cooling medium which flows through the reaction zone via one or more helical patterns, each pattern containing one or more helices.

2. Process according to claim 1, wherein the cooling medium flows through two or more concentric helical patterns.

3. Process according to claim 2, wherein the screw direction of adjacent helical patterns are opposite to each other.

4. Process according to claim 2 or 3, wherein an increasing number of helices is used in the helical patterns which are situated at a larger distance from the centre.

5. Process according to claim 1, wherein a cylindrical reaction vessel is used provided with one or more helical wound tubes or tube bundles, each bundle comprising two or more helical wound tubes of substantially the same dimensions and situated in a concentric ring or a number of concentric rings around the central axis of the reaction vessel.

6. Process according to one or more of the claims 1-5 which is carried out at a temperature in the range of from 200 to 285 °C, a total pressure in the range of from 1 to 60 bar abs. and a gas hourly space velocity in the range of from 2000 to 8000 h⁻¹.

7. Process according to one or more of the claims 1-6 in which a catalyst is used comprising 2-25% by weight of silver and 50-5000 parts per million of an alkali metal promoter, based on the weight of the total catalyst.

8. Process according to claim 7 wherein the alkali metal is cesium.

9. Process according to one or more of the claims 1-6 in which a catalyst is used comprising 2-25% by weight of silver, from 0.01 to 15 millimol of rhenium and from 10 to 3000 parts per million of alkali metal, all based on 1 kg of catalyst.


## Patentansprüche

1. Verfahren zur Herstellung von Ethylenoxid durch Umsetzung von Ethylen mit molekularem Sauerstoff in Gegenwart eines silberhaltigen Katalysators, umfassend das Durchleiten eines Gasgemisches aus Ethylen und Sauerstoff durch eine Reaktionszone, enthaltend die Katalysatorteilchen, während Wärme aus der Reaktionszone abgezogen wird durch ein Kühlmedium, das durch die Reaktionszone über eine oder mehrere helixförmige Anordnungen fließt, wobei jede Anordnung eine oder mehrere Helices umfaßt.

2. Verfahren nach Anspruch 1, wobei das Kühlmedium durch zwei oder mehrere konzentrische helixförmige Anordnungen fließt.

3. Verfahren nach Anspruch 2, wobei die Schraubenrichtung der benachbarten helixförmigen Anordnungen entgegengesetzt ist.

4. Verfahren nach Anspruch 2 oder 3, wobei eine zunehmende Zahl von Helices in den helixförmigen Anordnungen angewandt wird, die sich in einem größeren Abstand vom Zentrum befinden.

5. Verfahren nach Anspruch 1, wobei ein zylinderförmiges Reaktionsgefäß angewandt wird, das mit einem oder mehreren helixförmig gewundenen Rohren oder Rohrbündeln versehen ist, wobei jedes Rohrbündel zwei oder mehrere helixförmig gewundene Rohre mit im wesentlichen den gleichen Dimensionen umfaßt, die sich in einem konzentrischen Ring befinden, oder eine Anzahl konzentrischer Ringe um die Mittelachse des Reaktionsgefäßes.

6. Verfahren nach einem der mehreren der Ansprüche 1 bis 5, das bei einer Temperatur im Bereich von 200 bis 285°C, einem Gesamtdruck im Bereich von 1 bis 60 bar abs. und einer Raumgeschwindigkeit des Gases im Bereich von 2000 bis 8000 h⁻¹ durchgeführt wird.

7, Verfahren nach einem oder mehreren der Ansprüche 1 bis 6, wobei ein Katalysator angewandt wird, umfassend 2 bis 25 Gew.-% silber und 50 bis 5000 ppm eines Alkallmetallpromotors, bezogen auf das Gewicht des Gesamtkatalysators.

8. Verfahren nach Anspruch 7, wobei das Alkalimetall Cäsium ist.

9. Verfahren nach einem oder mehreren der Ansprüche 1 bis 6, wobei ein Katalysator angewandt wird, umfassend 2 bis 25 Gew.-% Silber, 0,01 bis 15 mmol Rhenium und 10 bis 3000 ppm Alkali-metall, jeweils bezogen auf 1 kg Katalysator.


## Revendications

1. Un procédé pour la préparation d'oxyde d'éthylène en faisant réagir de l'éthylène avec de l'oxygène moléculaire en présence d'un catalyseur renfermant de l'argent, qui consiste à faire passer un mélange gazeux d'éthylène et d'oxygène à travers une zone de réaction renfermant les particules de catalyseur, tout en éliminant de la chaleur de la zone de réaction par un milieu de refroidissement qui s'écoule à travers la zone de réaction par l'intermédiaire d'un ou de plus d'un dispositif hélicoïdal, chaque dispositif renfermant une ou plus d'une hélice.

2. Procédé selon la revendication 1, dans lequel le milieu de refroidissement s'écoule à travers deux ou plus de deux dispositifs concentriques hélicoïdaux.

3. Procédé selon la revendication 2, dans lequel la direction des hélices des dispositifs hélicoïdaux adjacents sont opposées l'une à l'autre.

4. Procédé selon la revendication 2 ou 3, dans lequel un nombre croissant d'hélices est utilisé dans les dispositifs hélicoïdaux qui sont situés à une distance plus grande du centre.

5. Procédé selon la revendication 1, dans lequel on utilise un récipient cylindrique pourvu d'un ou de plus d'un tube ou faisceau de tubes, enroulé en hélice, chaque faisceau comportant deux ou plus de deux tubes enroulés en hélice, présentant pratiquement les mêmes dimensions et disposés dans un anneau concentrique ou bien un certain nombre d'anneaux concentriques autour de l'axe du récipient de réaction.

6. Procédé selon une ou plus d'une des revendications 1 à 5, qui est réalisé à une température dans la gamme de 200 à 285°C, sous une pression totale dans la gamme de 1 à 60 bars absolus et une vitesse spatiale horaire du gaz dans la gamme de 2000 à 8000 h⁻¹.

7. Procédé selon l'une ou plus d'une des revendications 1 à 6, dans lequel on utilise un catalyseur comportant de 2 à 25 % en poids d'argent et de 50 à 5 000 parties par million d'un promoteur à base de métal alcalin, en se basant sur le poids du catalyseur total.

8. Procédé selon la revendication 7, dans lequel le métal alcalin est le césium.

9. Procédé selon une ou plus d'une des revendications 1 à 6, dans lequel on utilise un catalyseur comportant de 2 à 25 % en poids d'argent, de 0,01 à 15 mmoles de rhénium et de 10 à 3000 parties par million de métal alcalin, en se basant à chaque fois sur un kilo de catalyseur.